# EUROPÄISCHE PATENTSCHRIFT

(11) **EP 0 857 058 B1**
(45) Veröffentlichungstag und Bekanntmachung des Hinweises auf die Patenterteilung: **19.09.2001**
(21) Anmeldenummer: 96934630.3
(22) Anmeldetag: 11.10.1996
(51) Int. Cl.: A61K 7/42, G03C 1/815

(54) **SUBSTITUIERTE BENZYLIDENCYANESSIGESTER**
SUBSTITUTED BENZYLIDENECYANOACETIC ACID ESTERS
ESTERS BENZYLIDENECYANACETIQUES SUBSTITUES

(30) Priorität: 20.10.1995 DE 19539189
(43) Veröffentlichungstag der Anmeldung: 12.08.1998
(73) Patentinhaber: BASF AKTIENGESELLSCHAFT, 67056 Ludwigshafen (DE)
(72) Erfinder: TRENTMANN, Beate, D-68307 Mannheim (DE)
(86) Internationale Anmeldenummer: EP9604417
(87) Internationale Veröffentlichungsnummer: WO9715279

(56) Entgegenhaltungen:
- EP-A- 0 005 182
- DE-A- 1 443 920
- DE-A- 2 049 289
- DE-B- 1 087 902
- CHEMICAL ABSTRACTS, vol. 71, no. 23, 8.Dezember 1969 Columbus, Ohio, US; abstract no. 112226, XP002025738 & JOURNAL OF PHYS. CHEM., Bd. 73, Nr. 10, Seiten 3370-3, "magnetic resonance studies..."

## Beschreibung

Die vorliegende Erfindung betrifft substituierte Benzylidencyanessigester der Formel I, 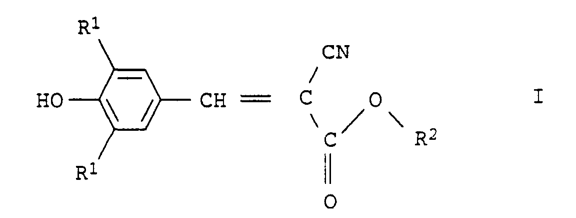 deren Verwendung als Lichtschutzmittel, deren Verwendung in kosmetischen Präparaten sowie kosmetische Mittel, enthaltend diese Verbindungen.

Lichtschutzmittel auf der Basis von substituierten Benzylidencyanessigestern sind bekannt.

In BE 757 036 wird unter anderem die Verbindung 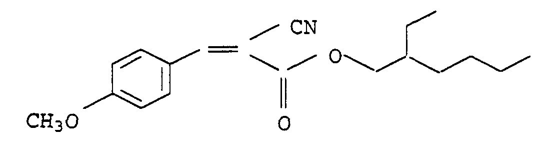 als lichtempfindliches Photomaterial beschrieben.

In DE 10 87 902 werden Kondensationsprodukte aus Benzaldehyden mit aktiven Methylengruppen enthaltenden Verbindungen beschrieben. Unter vielen anderen Verbindungen werden auch Kondensationsprodukte von 4-Oxy-3,5-di-t-butylbenzaldehyd mit Malonsäurediethylester, Cyanessigester, Malondinitril oder Malonsäure genannt (Seite 1, zweite Spalte, Gruppe VI). Diese Verbindungen werden als geeignete Lichtschutzmittel für Filme, Folien, Fasern und Fäden beschrieben.

In DE 28 16 819 werden substituierte Benzylidencyanessigester der folgenden Struktur als UV-A Filter beschrieben: 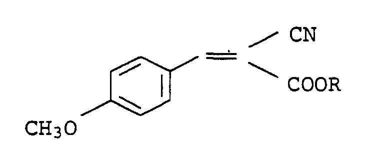 wobei festgestellt wird, daß hinsichtlich möglicher Substitutionen am Aromaten die para-Monosubstitution das Optimum darstellt und hier wiederum der Methoxyrest optimale Eigenschaften verleiht. Bezüglich des Alkoholrestes R wird festgestellt, daß Verbindungen mit R=n-Hexyl, n-Octyl, n-Decyl, iso-Nonyl, und iso-Decyl am besten geeignet sind.

Da kosmetische Lichtschutzmittel neben den "Photoeigenschaften" wie geeignetes Absorptionsmaximum, hohe spezifische Extinktion, Photostabilität, eine Reihe weiterer "anwendungstechnischer Eigenschaften" wie gute Öllöslichkeit, pH-Stabilität, Oxidationsstabilität, Thermostabilität, möglichst keine Eigenfärbung und kein Eigengeruch erfüllen müssen und daneben auch toxikologisch unbedenklich sein müssen, bestand die Aufgabe, die bisher bekannten Produkte hinsichtlich der o.g. Eigenschaften zu optimieren.

Es wurde nun gefunden, daß Verbindungen der Formel I 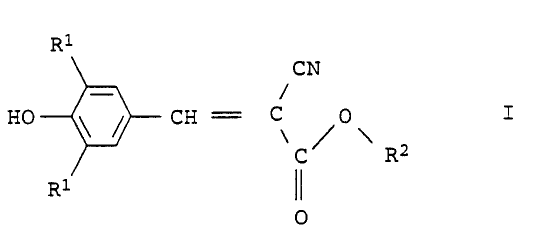 worin
- R¹=: i-Propyl, i-Butyl oder t-Butyl,
- R²=: Alkyl mit 6-14 C-Atomen, 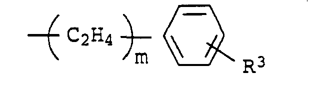 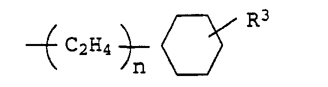 mit R³ = H oder C₁-C₄-Alkyl
und m, n = 0 oder 1 bedeuten, hinsichtlich vieler der o.g. Erfordernisse, vor allem hinsichtlich der Photostabilität, bessere Eigenschaften besitzen als die aus dem Stand der Technik bekannten Verbindungen.

Als besonders geeignetes Lichtschutzmittel haben sich Verbindungen der Formel I herausgestellt, bei denen beide Reste R¹ = tert.-Butyl und R² einen verzweigten Alkylrest mit 8-12 C-Atomen oder -C₂H₄-C₆H₅ oder 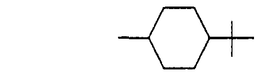 bedeuten.

R² kann dabei z.B. den Rest 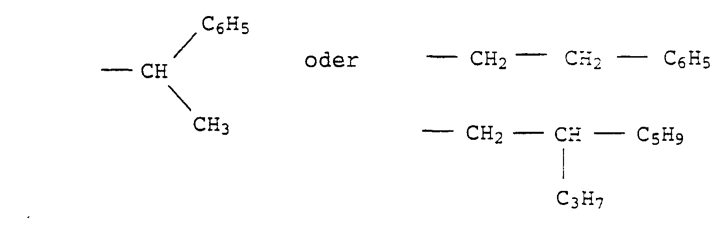 oder bedeuten.

Insbesondere vorteilhaft ist eine Verbindung der Formel I, in der beide Reste R¹ = tert.-Butyl und R² = 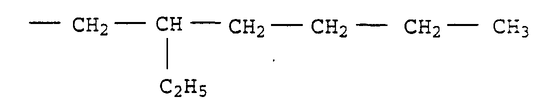 bedeuten.

Die erfindungsgemäßen Verbindungen lassen sich in an sich bekannter Weise in einer Knoevenagelkondensation aus den entsprechenden Benzaldehyden und Cyanessigestern herstellen (s. z.B. Organikum, Ausgabe 1988, S. 459). Die entsprechenden Cyanessigestern wurden durch Umesterung eines kommerziell erhältlichen Cyanessigesters mit dem entsprechenden Alkohol in an sich bekannter Weise hergestellt.

Die erfindungsgemäßen Verbindungen eignen sich insbesondere als Lichtschutzmittel für Materialien, die durch UV-Strahlung angegriffen werden, beispielsweise Fäden, Fasern, Folien, Filme und andere Formkörper aus Kunststoffen.

Besonders gut geeignet sind die erfindungsgemäßen Verbindungen zum Schutz der menschlichen Haut vor UV-Strahlung. Sie können in vielerlei kosmetischen und medizinischen Präparaten wie Sonnenöle, Sonnencremes, Sonnenmilch, Sonnengelen, Lippenstift, Hautcremes, Haargelen und fettfreien Gelen eingesetzt werden.

### Beispiele

### Beispiel 1

### 3,5 Di-tertiär-butyl-4-hydroxybenzylidencyanessigsäure-2-phenylethylester

5,9 g 3,5 Di-tertiär-butyl-4-hydroxybenzaldehyd wurden in 50 ml Toluol gelöst.

4,7 g Cyanessigsäure 2-phenylethylester, 0,1 g Piperidin und 0,25 g Essigsäure wurden auf Rückfluß erhitzt. In 2 h wurden 0,4 g H₂O ausgekreist. Es wurde abgekühlt, mit Wasser gewaschen, mit Natriumhydrogencarbonatlösung gewaschen, getrocknet und eingeengt. Der Rohaustrag wurde umkristallisiert.
Ausbeute: 9,9 g (98 %).

### Beispiel 2

### 3,5-Di-tertiär-butyl-4-hydroxybenzylidencyanessigsäure-2-ethylhexylester

28,1 g 3,5-Di-tertiär-butyl-4-hydroxybenzaldehyd wurden in 60 ml Toluol gelöst. Es wurden 21,7 g Cyanessigsäure- 2-ethylhexylester, 0,27 g Piperidin und 0,67 g Essigsäure zugegeben. Man erhitzte auf Rückfluß und kreiste ca. 2 g Wasser aus. Die klare Lösung wurde gewaschen, getrocknet und eingeengt. Ausbeute: 46,2 g hellgelbes Öl (93 %).

### Beispiel 3

### 3,5-Di-tertiär-butyl-4-hydroxybenzylidencyanessigsäure-4-tertiärbutylcyclohexylester

5,4 g 3,5-Di-tertiär-butyl-4-hydroxy-benzaldehyd wurden in 50 ml Toluol gelöst. 5,9 g Cyanessigsäure- 4-tertiär-butylcyclohexylester, 0,1 g Piperidin und 0,25 g Essigsäure wurden zugegeben. Am Rückfluß wurden 0,4 g Wasser ausgekreist. Es wurde gewaschen, getrocknet und eingeengt.
Ausbeute: 10,6 g (96 %) Kristalle

| Eigenschaften: | | | | |
|---|---|---|---|---|
| Beispiel | λₘₐₓ [nm] | E₁¹ | Löslichkeit | Photostabilität |
| 1 | 356 | 484 | gut | 98 % |
| 2 | 357 | 638 | sehr gut | 99 % |
| 3 | 355 | 623 | gut | 92 % |

Die Löslichkeit wurde durch Auflösung der Substanzen bei Raumtemperatur in C₁₂-C₁₅-Alkyl-Benzoaten bestimmt.

Die Photostabilität wurde bestimmt durch Bestrahlen einer Lösung der entsprechenden Verbindung mit einem Sun-Test-Gerät der Fa. Heräus. Bestrahlungszeit 30 min. Angegeben ist der noch vorhandene Anteil der Verbindung in Prozent der Ausgangsmenge.

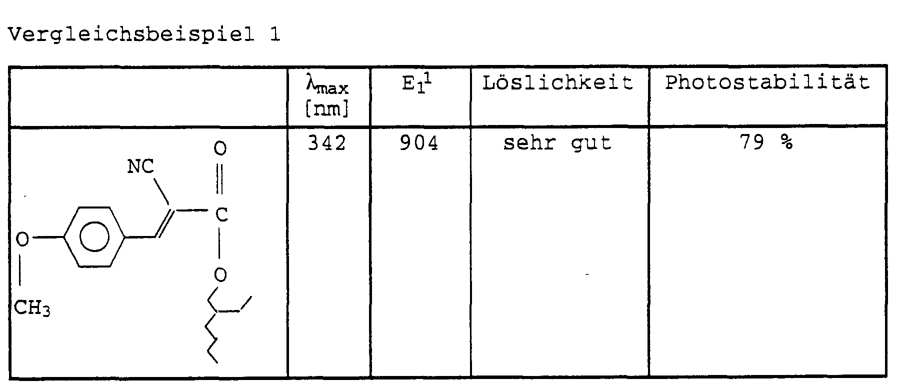

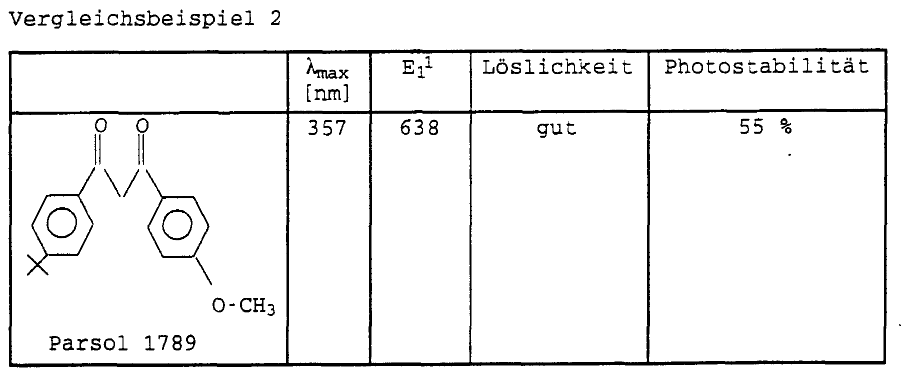

Bei Parsol 1789 handelt es sich um ein zugelassenes Marktprodukt (UV-A Filter).

Es zeigt sich, daß die erfindungsgemäßen Verbindungen, insbesondere in der wichtigen Eigenschaft der Photostabilität, überraschende Vorteile gegenüber einer strukturell ähnlichen, bekannten Verbindung sowie gegenüber einem zugelassenem Marktprodukt aufweisen.

### Anwendungstechnische Beispiele

Im folgenden wird die Zusammensetzung kosmetischer Mittel angegeben, in denen sich die erfindungsgemäßen Verbindungen besonders vorteilhaft einsetzen lassen.

Allgemeine Herstellvorschrift zur Herstellung von Emulsionen für kosmetische Zwecke:

Alle öllöslichen Bestandteile in einem Rührkessel auf 85°C erwärmen.

Wenn alle Bestandteile geschmolzen sind, bzw. als Flüssigphase. vorliegen, wird die Wasserphase unter Homogenisieren eingearbeitet.

Unter Rühren wird die Emulsion auf ca. 40°C abgekühlt, parfümiert, homogenisiert und dann unter ständigem Rühren auf 25°C abgekühlt.

| Zusammensetzung für die Lippenpflege | |
|---|---|
| ad 100 | Eucerinum anhydricum |
| 10,00 | Glycerin |
| 10,00 | Titandioxid |
| 0,5-10 | Verbindung aus Beispiel 1 |
| 8,00 | Octyl Methoxycinnamat |
| 5,00 | Zinkoxid |
| 4,00 | Castor Öl |
| 4,00 | Pentaerythrityl Stearat/Caprat/Caprylat Adipat |
| 3,00 | Glyceryl Stearat SE |
| 2,00 | Bienenwachs |
| 2,00 | Microcrystallines Wachs |
| 2,00 | Quaternium-18 Bentonit |
| 1,50 | PEG-45/Dodecyl Glycol Copolymer |

| Zusammensetzung für Sunblocker mit Mikropigmenten | |
|---|---|
| ad 100 | Wasser |
| 10,00 | Parsol MCX Octyl Methoxycinnamat |
| 6,00 | PEG-7-Hydrogenated Castor Öl |
| 6,00 | Titandioxid |
| 0,5-10 | Verbindung aus Beispiel 1 |
| 5,00 | Mineralöl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Butyl Methoxydibenzoylmethan |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

| Fettfreies Gel | |
|---|---|
| ad 100 | Wasser |
| 8,00 | Octyl Methoxycinnamat |
| 7,00 | Titanium Dioxid |
| 0,5-10 | Verbindung nach Beispiel 2 |
| 5,00 | Glycerin |
| 5,00 | PEG-25 PABA |
| 1,00 | 4-Methylbenzyliden Campher |
| 0,40 | Acrylate C10-C30 Alkyl Acrylat Copolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

| Sonnencreme (LSF 20) | |
|---|---|
| ad 100 | Wasser |
| 8,00 | Octyl Methoxycinnamat |
| 8,00 | Titandioxid |
| 6,00 | PEG-7-Hydrogenated Castor Öl |
| 0,5-10 | Verbindung nach Beispiel 2 |
| 6,00 | Mineralöl |
| 5,00 | Zinkoxid |
| 5,00 | Isopropyl Palmitat |
| 5,00 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

| Sonnencreme wasserfest | |
|---|---|
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 0,5-10 | Verbindung nach Beispiel 2 |
| 4,00 | Glycerin |
| 3,00 | Jojoba Öl |
| 2,00 | 4-Methylbenzyliden Campher |
| 2,00 | Titanium Dioxid |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

| Sonnenmilch (LSF 6) | |
|---|---|
| ad 100 | Wasser |
| 10,00 | Mineralöl |
| 6,00 | PEG-7-Hydrogenated Castor Öl |
| 5,00 | Isopropyl Palmitat |
| 3,50 | Octyl Methoxycinnamat |
| 0,5-10 | Verbindung nach Beispiel 2 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 0,30 | Glycerin |
| 0,25 | Methylparaben |
| 0,15 | Propylparaben |
| 0,05 | Tocopherol |

## Patentansprüche

1. Verbindung der Formel I 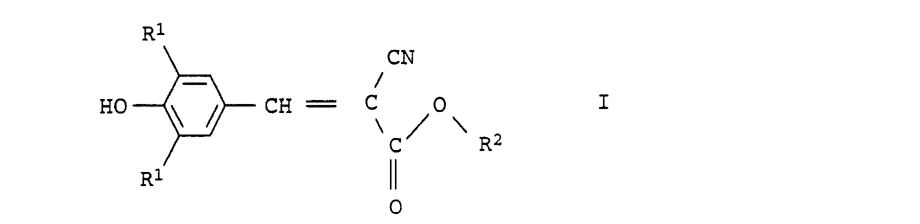 worin
R¹= i-Propyl, i-Butyl oder t-Butyl,
R²= Alkyl mit 6-14 C-Atomen, 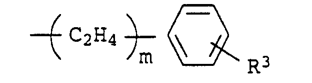 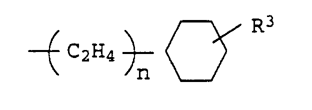 mit R³ = H oder C₁-C₄-Alkyl
und m, n = 0 oder 1
bedeuten.

2. Verbindungen der Formel I nach Anspruch 1, in denen
R¹ t-Butyl und
R² einen verzweigten Alkylrest mit 8-12 C-Atomen, -C₂H₄-C₆H₅ oder 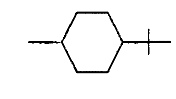
bedeuten.

3. Verbindungen der Formel I nach Anspruch 1, in der
R¹= t-Butyl und
R²= 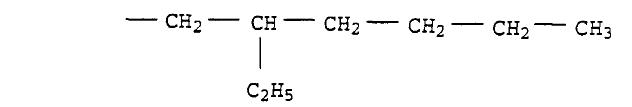 oder
―CH₂―CH₂―C₆H₅
bedeuten.

4. Kosmetische Verwendung der Verbindungen gemäß Anspruch 1 als Lichtschutzmittel.

5. Kosmetische Verwendung der Verbindungen gemäß Anspruch 1 in kosmetischen Präparaten.

6. Kosmetische Mittel, die als Lichtschutzmittel eine Verbindun nach Anspruch 1 allein oder zusammen mit anderen UV-A oder UV-B Filtern enthalten.

## Claims

1. A compound of the formula I 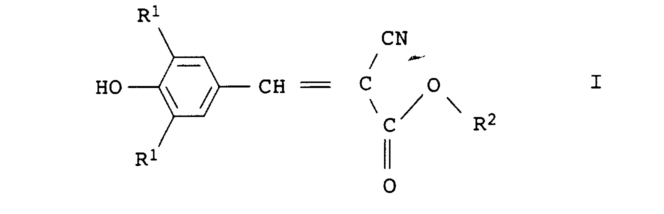 where
R¹ is i-propyl, i-butyl or t-butyl,
R² is alkyl with 6-14 carbon atoms, 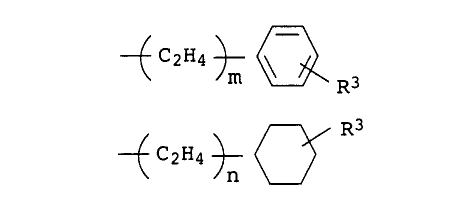 with R³ = H or C₁-C₄-alkyl
and m, n = 0 or 1.

2. A compound of the formula I as claimed in claim 1, where
R¹ is t-butyl and
R² is a branched alkyl radical with 8-12 carbon atoms, -C₂H₄-C₆H₅ or

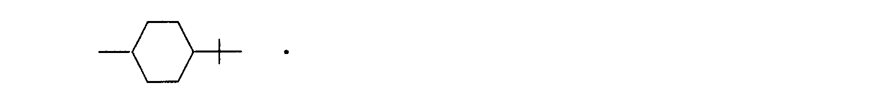

3. A compound of the formula I as claimed in claim 1, where
R¹ is t-butyl and
R² is 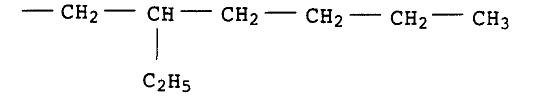 or
― CH₂ ― CH₂ ― C₆H₅.
The cosmetic use of a compound as claimed in claim 1 as sunscreen agent or light stabilizer.
The cosmetic use of a compound as claimed in claim 1 in cosmetic products.
A cosmetic composition which comprises as sunscreen agent a compound as claimed in claim 1 alone or together with other UV-A or UV-B filters.

## Revendications

1. Composé de formule I 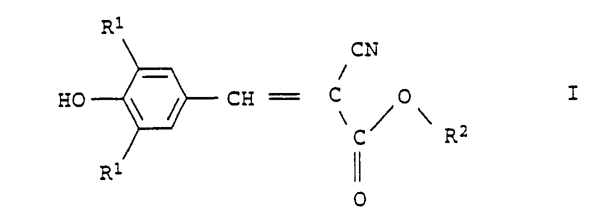 où
R¹ = isopropyle, isobutyle ou tert-butyle,
R² = alkyle ayant 6-14 atomes de carbone, 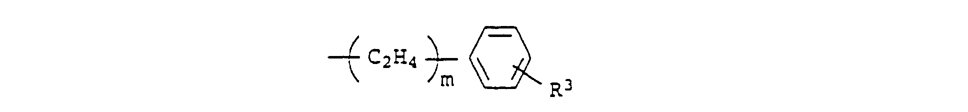 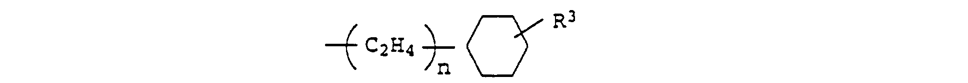 avec R³ = H ou alkyle en C₁-C₄
et m, n = 0 ou 1.

2. Composés de formule I selon la revendication 1, dans lesquels
R¹ représente un groupe tert-butyle et
R² désigne un reste alkyle ramifié ayant 8-12 atomes de carbone, -C₂H₄-C₆H₅
ou

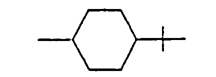

3. Composés de formule I selon la revendication 1, dans laquelle
R¹ = tert-butyle et
R² = 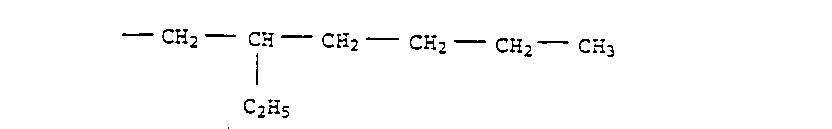
ou
―CH₂―CH₂―C₆H₅

4. Utilisation cosmétique des composés selon la revendication 1, en tant qu'agents de protection contre la lumière.

5. Utilisation cosmétique des composés selon la revendication 1 dans des préparations cosmétiques.

6. Produits cosmétiques, qui contiennent en tant qu'agent de protection contre la lumière un composé selon la revendication 1 seul ou combiné avec d'autres filtres UV-A ou UV-B.
